# EUROPEAN PATENT APPLICATION

(11) **EP 4 696 257 A1**
(43) Date of publication of application: **18.02.2026**
(21) Application number: 25193848.6
(22) Date of filing: 04.08.2025
(51) Int. Cl.: A61B 17/34

(54) **SURGICAL ACCESS DEVICE WITH IMPROVED SEAL HOUSING AND TROCAR INTERFACE**

(30) Priority: 12.08.2024 WO PCT/CN2024/111378
(71) Applicant: Covidien LP, Mansfield, MA 02048 (US)
(72) Inventor: Jin, Tingting, Shanghai (CN)
(74) Representative: Gray, James

(57) **Abstract**

A surgical access device includes a trocar assembly and a seal housing assembly that is releasably coupled to the trocar assembly. The trocar assembly supports a release mechanism that facilitates attachment and detachment of the seal housing assembly to/from the trocar assembly in a fast and efficient manner.

## Description

### CROSS-REFERENCE TO RELATED APPLICATION

This application claims the benefit of PCT Patent Application Serial No. PCT/CN2024/111378, filed August 12, 2024, the entire content of which is incorporated herein by reference.

### FIELD

This disclosure generally relates to surgical instruments for accessing a surgical site. More specifically, this disclosure relates to a surgical access device with an improved seal housing and trocar interface.

### BACKGROUND

In minimally invasive surgical procedures, including endoscopic and laparoscopic surgeries, a surgical access device permits introduction of a variety of surgical instruments into a body cavity. The surgical access device includes a cannula or access port that is introduced through an opening in tissue (e.g., a naturally occurring orifice or an incision) to provide access to an underlying surgical site in the body. The opening is typically made using an obturator having a blunt or sharp tip that may be inserted through a passageway of the surgical access device. The cannula includes a tube of rigid material with a thin wall construction through which surgical instruments can be introduced to perform the minimally invasive surgical procedure.

The surgical access device typically includes a trocar assembly and a seal housing that are coupled together. The trocar assembly includes a tubular body or cannula and a seal housing that is releasably coupled to the trocar housing. The cannula provides a tubular passageway that is configured to facilitate passage of surgical instrumentation. The seal housing includes one or more seals that engage the surgical instrumentation to prevent the flow of insufflation gases from within the body cavity during the surgical procedure. After a surgical procedure is completed, the seal housing can be removed from the trocar assembly to facilitate removal of tissue from the body cavity through the cannula.

A continuing need exists in the art for a surgical access device that renders the seal housing to be quickly and easily attached to and removed from the trocar assembly.

### SUMMARY

A surgical access device includes a trocar assembly and a seal housing assembly that is releasably coupled to the trocar assembly. The trocar assembly supports a release mechanism that facilitates attachment and detachment of the seal housing assembly to/from the trocar assembly in a fast and efficient manner.

Aspects of this disclosure are directed to a surgical access device including a trocar assembly, a release mechanism, and a seal housing assembly. The trocar assembly includes a cannula, a distal housing portion, a proximal housing portion, and a first seal member. The cannula defines a longitudinal axis and a channel that extends through the cannula. The proximal housing portion has an annular configuration and defines openings that extend through the proximal housing portion. The distal housing portion defines a first cavity that communicates with the channel in the cannula, and the first seal member is received within the first cavity between the distal housing portion and the proximal housing portion. The release mechanism is supported on the trocar assembly between the distal housing portion and the proximal housing portion and includes a release member and a biasing member. The release member includes a first leg and a second leg, and each of the first leg and the second leg defines a cam surface. The release member is movable in a direction transverse to the longitudinal axis between a locked position and an unlocked position. In the locked position, the cam surfaces of the first leg and the second leg are positioned beneath the openings in the proximal housing portion of the trocar assembly. The seal housing assembly includes a proximal housing portion, a distal housing portion, and a seal assembly. The proximal housing portion and the distal housing portion of the seal housing assembly define a second cavity, and the seal assembly is received within the second cavity. The distal housing portion includes fingers, and each of the fingers is received within one of the openings of the proximal housing portion of the trocar assembly. Receipt of the fingers within the openings causes the release member to move from the locked position to the unlocked position.

Other aspects of this disclosure are directed to a trocar assembly and a release mechanism. The trocar assembly includes a cannula, a distal housing portion, a proximal housing portion, and a seal member. The cannula defines a longitudinal axis and a channel that extends through the cannula. The proximal housing portion has an annular configuration and defines openings that extend through the proximal housing portion. The distal housing portion defines a first cavity that communicates with the channel in the cannula, and the seal member is received within the first cavity between the distal housing portion and the proximal housing portion. The release mechanism is supported on the trocar assembly between the distal housing portion and the proximal housing portion and includes a release member and a biasing member. The release member includes a first leg and a second leg, and each of the first leg and the second leg defines a cam surface. The release member is movable in a direction transverse to the longitudinal axis between a locked position and an unlocked position. In the locked position, the cam surfaces of the first leg and the second leg are positioned beneath the openings in the proximal housing portion of the trocar assembly.

In aspects of the disclosure, the biasing member is positioned to urge the release member towards the locked position.

In some aspects of the disclosure, each of the first and second legs defines a slot that is configured to allow passage of the fingers when the release member is in the unlocked position

In certain aspects of the disclosure, the cam surface of each of the first leg and the second leg of the release member define a portion of one of the slots.

In aspects of the disclosure, the release member includes a back span that connects the first leg to the second leg.

In some aspects of the disclosure, the back span extends outwardly of the proximal housing portion of the trocar assembly.

In certain aspects of the disclosure, the proximal housing portion of the trocar assembly includes guide members and each of the first leg and the second leg defines a recess, and the guide members are received within the recesses to guide movement of the release member between the locked position and the unlocked position.

In aspects of the disclosure, the seal assembly includes a second seal member that is formed of a resilient material and defines an opening for receiving a surgical instrument.

In some aspects of the disclosure, the seal assembly is movable within the second cavity in a direction transverse to the longitudinal axis.

In certain aspects of the disclosure, the seal assembly includes an annular body, and the second seal member is supported within the annular body.

In aspects of the disclosure, the annular body supports resilient strips that extend outwardly of the annular body and engage an inner wall of the proximal housing portion of the seal housing assembly.

In some aspects of the disclosure, the proximal housing portion of the trocar assembly includes a post, and the biasing member is a coil spring positioned about the post between the release member and the proximal housing portion.

Other features of the disclosure will be appreciated from the following description.

### BRIEF DESCRIPTION OF THE DRAWINGS

Various aspects of the disclosed surgical stapling device are described herein below with reference to the drawings, wherein:
FIG. 1 is a side perspective view of a surgical access device according to aspects of the disclosure including a trocar assembly and a seal housing assembly with the seal housing assembly coupled to the trocar housing;
FIG. 2 is a perspective view of the surgical access device shown in FIG. 1 with the seal housing assembly separated from the trocar assembly;
FIG. 3 is an exploded, side perspective view of the trocar assembly shown in FIG. 1;
FIG. 4 is an exploded perspective view of the seal housing assembly of the surgical access device shown in FIG. 1;
FIG. 5 is an enlarged view of the indicated area of detail shown in FIG. 3;
FIG. 6 is a bottom perspective view of a proximal housing portion and release mechanism of the trocar assembly shown in FIG. 1 with parts separated;
FIG 7 is a side view of the surgical access device shown in FIG. 1 with the seal housing assembly separated from the trocar assembly;
FIG. 8 is a cross-sectional view taken along section line 8-8 of FIG. 7;
FIG. 9 is a cross-sectional view taken along section line 9-9 of FIG. 7;
FIG 10 is a side view of the surgical access device shown in FIG. 1 as the seal housing assembly is coupled to the trocar assembly;
FIG. 11 is a cross-sectional view taken along section line 11-11 of FIG. 10;
FIG 12 is a side view of the surgical access device shown in FIG. 1 with the seal housing assembly coupled to the trocar assembly;
FIG. 13 is a cross-sectional view taken along section line 13-13 of FIG. 12;
FIG. 14 is a bottom perspective view of the seal housing assembly and the proximal housing portion of the trocar assembly with the seal housing assembly coupled to the proximal housing portion; and
FIG. 15 is a cross-sectional view taken through a release button of the release mechanism of the surgical access device shown in FIG. 1.

### DETAILED DESCRIPTION

Aspects of the surgical access device will now be described in detail with reference to the drawings in which like reference numerals designate identical or corresponding elements in each of the several views. However, it is to be understood that disclosed aspects of the surgical stapling device are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

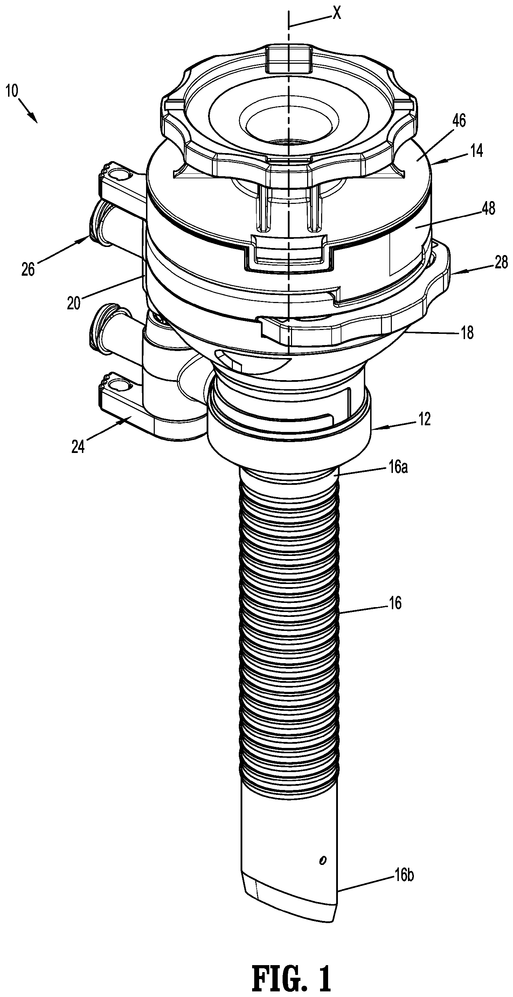

FIGS. 1-3 illustrate the disclosed surgical access device shown generally as access device 10. The access device 10 includes a trocar assembly 12 and a seal housing assembly 14 that is releasably coupled to the trocar assembly 12. The trocar assembly 12 includes a cannula 16, a distal housing portion 18, a proximal housing portion 20, a seal member 22, a first valve 24, a second valve 26, and a release mechanism 28. The cannula 16 has a proximal portion 16a secured to the distal housing portion 18 of the trocar assembly 12 and a distal portion 16b that is spaced from the distal housing portion 18. The cannula 16 may have a tubular configuration and defines a channel (not shown) that is dimensioned to facilitate passage of surgical instrumentation. In aspects of the disclosure, the cannula 16 can include an outer surface having annular ribs 30 that engage tissue to maintain the position of the cannula 16 within an opening in tissue. The distal portion 16b of the cannula 16 can have an angular configuration with a tapered distal edge 32 to facilitate entry into an opening or orifice.

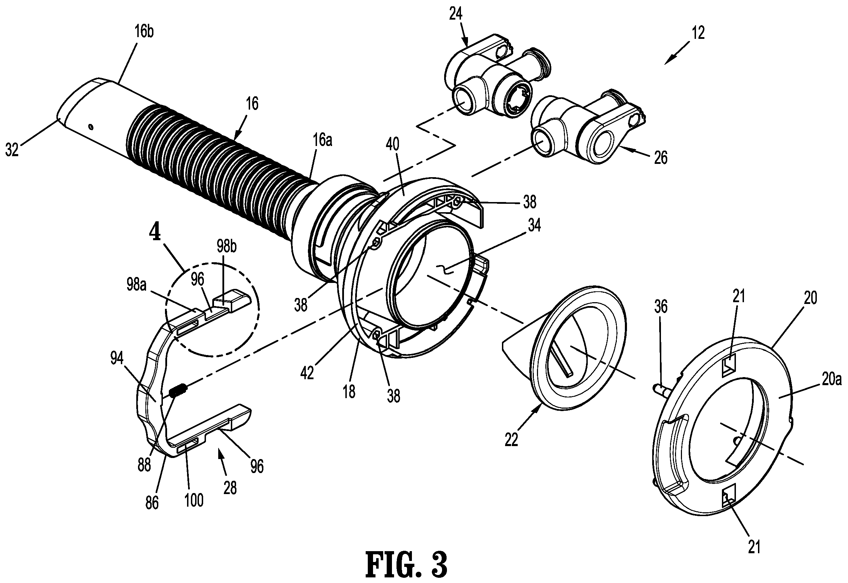

The distal housing portion 18 of the trocar assembly 12 is secured to the cannula 16 and defines a cavity 34 that is coaxial with the channel (not shown) that is defined by the tubular cannula 16. The proximal housing portion 20 of the trocar assembly 12 is secured to the proximal end of the distal housing portion 18 and has an annular configuration. In aspects of the disclosure, the proximal housing portion 20 includes pins 36 that are received within openings 38 defined in the distal housing portion 18 to secure the distal housing portion 18 to the proximal housing portion 20. The distal housing portion 18 includes a side wall 40 that defines a slot 42 (FIG. 3) that receives the release mechanism 28. It is envisioned that the slot 42 could be formed in the distal housing portion 18, the proximal housing portion 20, or both the distal housing portion 18 and the proximal housing portion 20. The proximal housing portion 20 also includes a proximal surface 20a that abuts the seal housing assembly 14 and defines diametrically positioned openings 21.

The seal member 22 is received within the cavity 34 of the distal housing portion 18 and is clamped between distal housing portion 18 and the proximal housing portion 20. In aspects of the disclosure, the seal member 22 is in the form of a duckbill valve and is movable between a closed position and an open position in response to insertion of a surgical instrument through the seal member.

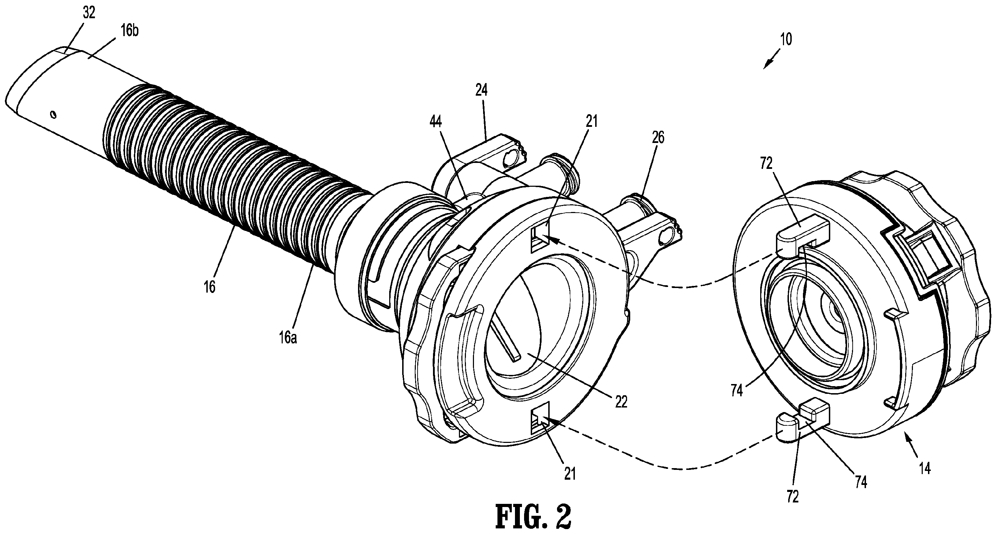

The first valve member 24 and the second valve member 26 are coupled to ports 44 (FIG. 2) (only one is shown) that communicate with the channel (not shown) defined by the cannula 16 and other passages within the trocar assembly 12 to deliver insufflation fluid and fluid to expand a balloon (not shown) as is known in the art.

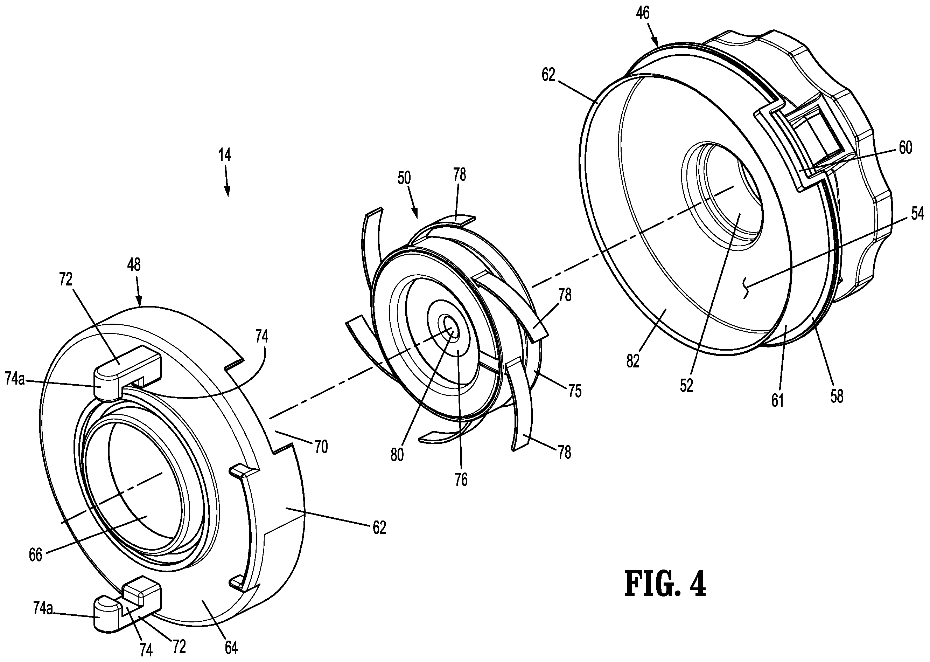

FIG. 4 illustrates the seal housing assembly 14 which includes a proximal housing portion 46, a distal housing portion 48, and a seal assembly 50. The proximal housing portion 46 defines a central through bore 52 and an annular cavity 54 that is positioned about the through bore 52 and includes a side wall 56. The side wall 56 includes an annular rib 58 that extends outwardly from the proximal housing portion 46 and forms a tab 60 that extends towards the distal housing portion 48. The annular rib 58 defines an annular recess 61 that is positioned at the distal end of the proximal housing portion 46.

The distal housing portion 48 has an annular configuration and includes a side wall 62 and a distal wall 64 that defines a through bore 66. The through bore 66 in the distal housing portion 48 is aligned with the through bore 52 in the proximal housing portion 46 along a longitudinal axis "X" (FIG. 1) of the trocar assembly 12. The distal housing portion 48 is coupled to the proximal housing portion 46 to cover the annular cavity 54. The annular cavity 54 receives the seal assembly 50. The side wall 62 of the distal housing portion 48 defines a rectangular cutout 70 that receives the tab 60 formed by the annular rib 58 to properly position the distal housing portion 48 in relation to the proximal housing portion 46. In aspects of the disclosure, the side wall 62 of the distal housing portion 48 is received within the annular recess 60 about the side wall 62 of the proximal seal housing 46. The distal wall of the distal housing portion 48 supports diametrically opposed fingers 72 that extend distally from the from the distal housing portion 48 towards the trocar assembly 12. Each of the fingers 72 defines a slot 74 that is positioned to receive a portion of the release mechanism 28 as described below. The distal end of each of the fingers 72 includes a curved cam surface 74a.

The seal assembly 50 is received within the cavity 54 defined between the distal housing portion 48 and the proximal housing portion of the seal housing assembly 14. In aspects of the disclosure, the seal assembly 50 includes an annular body 75, a seal member 76, a plurality of resilient strips 78. The seal member 76 is supported within the annular body 75 and defines an opening 80 for receiving a surgical instrument. The seal member 76 is formed of a resilient material which allows the opening 80 to expand to facilitate passage of the surgical instrument. The resilient strips 78 extend outwardly from the outer surface of the annular body 75 and are positioned to engage an inner wall 82 of the proximal housing portion of the seal housing assembly 14 to allow the seal assembly 50 to move transversely within the cavity 54. In that respect, the diameter of the annular body 75 is smaller than the diameter of the cavity 54, and the resilient strips 78 are configured to center the annular body 75 within the cavity 54. When the annular body 75 is centered within the cavity 54 of the seal housing assembly 14, the opening 80 is coaxial with the openings 52 and 66 formed in the proximal housing portion 46 and the distal housing portion 48, respectively, of the seal housing assembly 14.

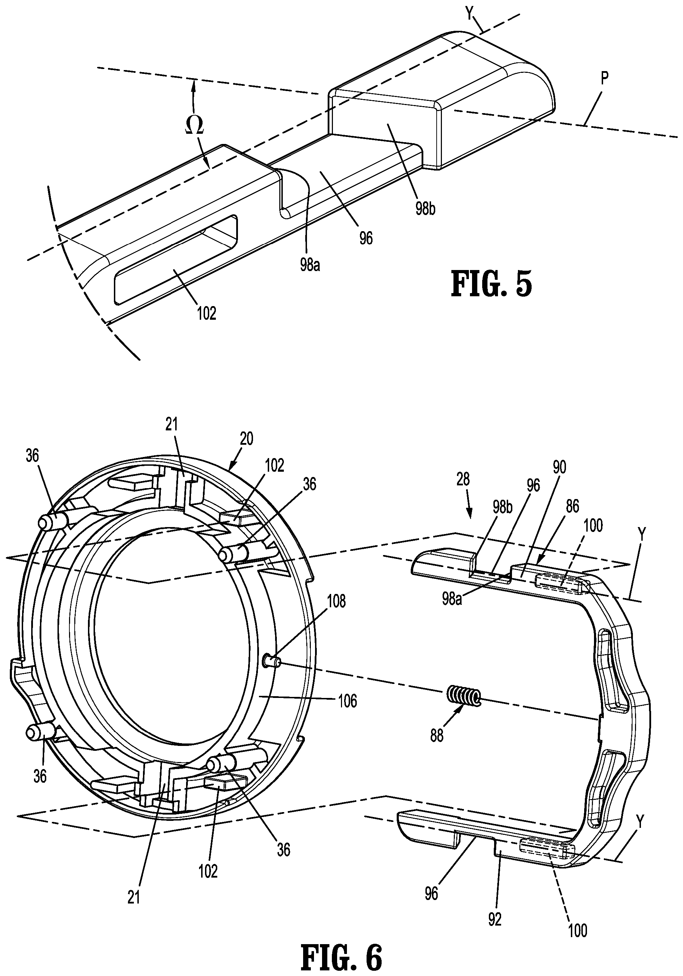

FIGS. 5 and 6 illustrate the release mechanism 28 which includes a release member 86 and a biasing member 88. The release member 86 is U-shaped and includes a first leg 90, a second leg 92, and a back span 94 that extends between the first leg 90 and the second leg 92. Each of the first and second legs 90 and 92 has a longitudinal axis "Y" (FIG. 5) and defines a slot 96 that is defined by a first slot wall 98a and a second slot wall 98b (FIG. 5). The second slot wall 98b forms a cam surface. In aspects of the disclosure, the first slot wall 98a defines a plane that is perpendicular to the longitudinal axis "Y" of the respective leg, and the second slot wall 98b defines a plane "P" that defines an acute angle Ω with the longitudinal axis "Y" of the respective leg. The back span 94 projects outwardly from the proximal housing portion 20 of the trocar assembly 12. The first leg 98a and the second leg 98b of the release member 86 each define a recess 100 that receives a guide member 102 that extends from an inner surface of the proximal housing portion 20 of the trocar assembly 12. The guide members 102 have a length that is smaller than the length of the recesses 100 to facilitate and guide movement of the release member 86 in relation to the proximal housing portion 20 of the trocar assembly 12 between a locked position and an unlocked position.

The biasing member 88 is positioned between the back span 94 of the release member 86 and an inner wall 106 of the proximal housing portion 20 of the trocar assembly 12. In aspects of the disclosure, the inner wall 106 supports a post 108 and the biasing member 88 is in the form of a coil spring that is received about the post 108. The biasing member 88 is positioned to urge the release member 86 outwardly from the proximal housing portion 20 towards the locked position.

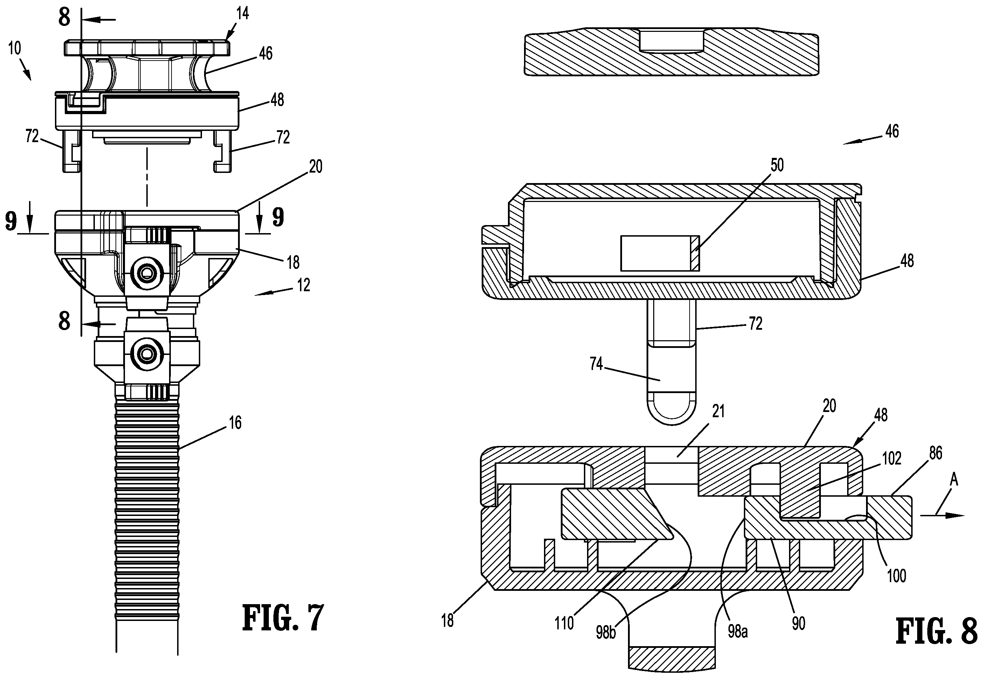

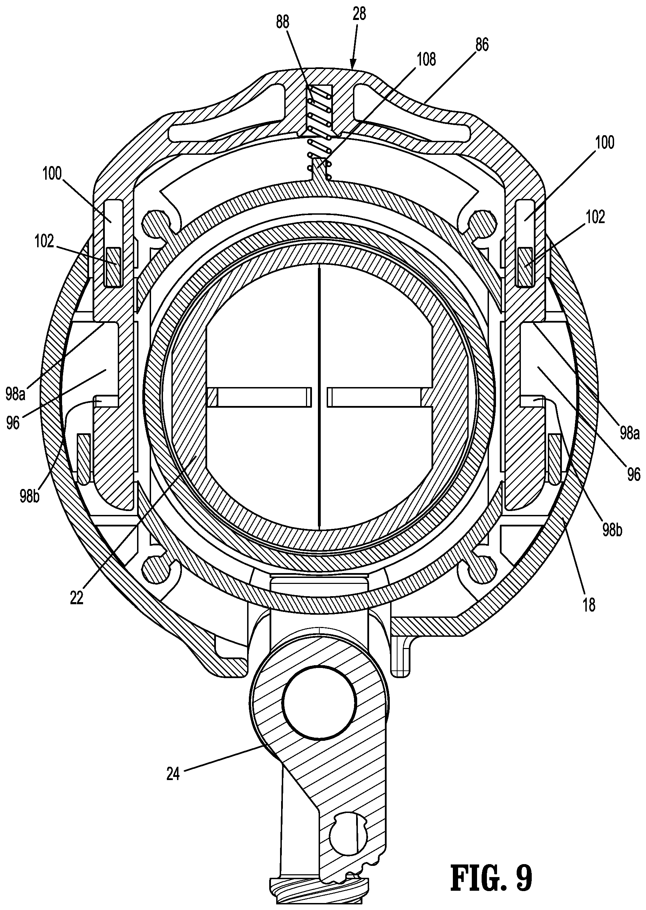

FIGS. 7-9 illustrate the trocar assembly 12 and the seal housing assembly 14 separated from each other. Prior to attachment of the seal housing assembly 14 to the trocar assembly 12, the biasing member 88 of the release mechanism 28 urges the release member 86 in the direction of arrow "A" in FIG. 8 to the locked position. In the locked position, the first leg 90 and the second leg 92 of the release member 86 are positioned between the distal housing portion 18 and proximal housing portion 20 with the second slot walls 98b of the release member 86 positioned beneath the openings 21 in the proximal housing portion 20 of the trocar assembly 12. As shown in FIG. 9, the guide members 102 of the proximal housing portion 20 of the trocar assembly 12 are positioned in one end of the recesses 100 formed in the release member 86.

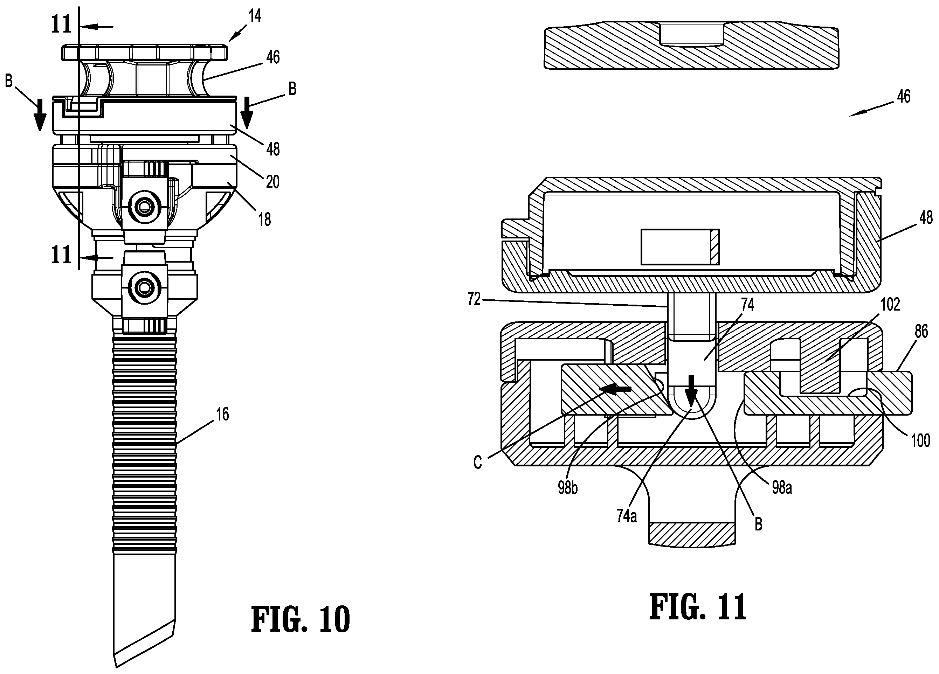

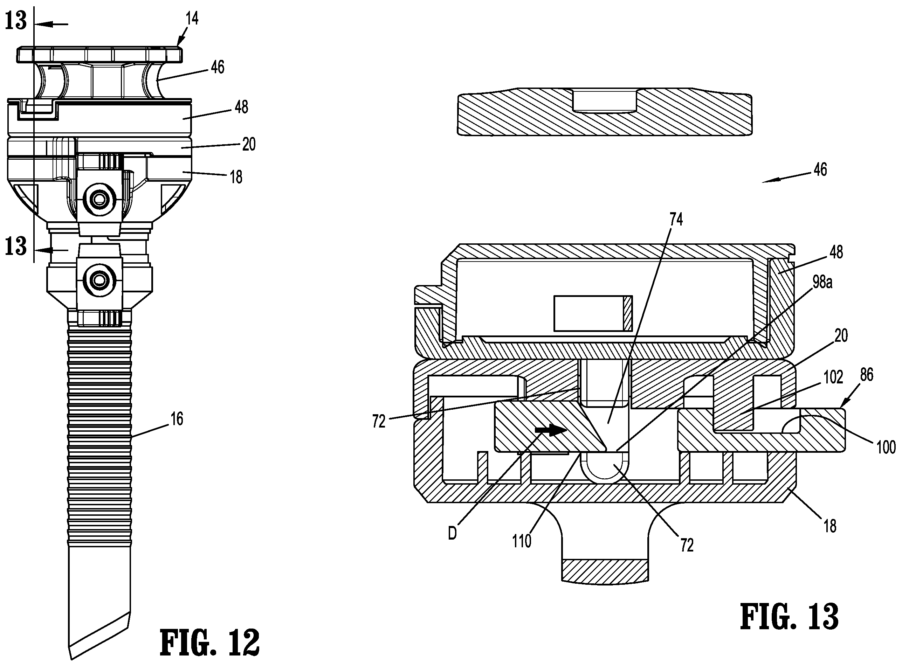

FIGS. 10 and 11 illustrate the access device 10 (FIG. 1) as the seal housing assembly 14 is attached to the trocar assembly 12. To attach the seal housing assembly 14 to the trocar assembly 12, the fingers 72 of the distal housing portion 48 of the seal housing assembly 14 are inserted into the openings 21 defined in the proximal housing portion 20 of the trocar assembly 12 in the direction indicated by arrow "B" in FIGS. 11 and 12. When the curved cam surface 74a of the fingers 72 engage the second slot walls 98b of the release member 86 and the fingers 72 move further in the direction of arrow "B", the release member 86 is moved from the locked position towards the unlocked position in the direction indicated by arrow "C" in FIG. 11 to the unlocked position. When the release member 86 moves to the unlocked position, the biasing member 88 of the release mechanism 28 is compressed.

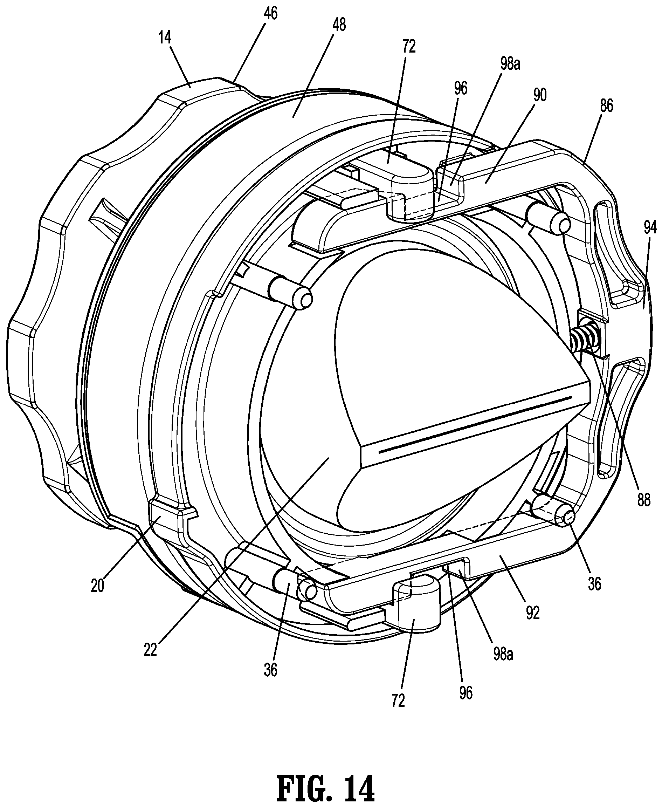

FIGS. 12-14 illustrate the access device 10 (FIG. 1) with the seal housing assembly 14 attached to the trocar assembly 12. When the fingers 72 of the distal housing portion 48 are moved to a position in which the slots 74 in the fingers 72 are aligned with the first leg 90 and the second leg 92 of the release member 86, the biasing member 88 returns the release member 86 to the locked position in the direction indicated by arrow "D" in FIG. 13. In the locked position, a portion 110 (FIG. 13) the first leg 90 and a portion (not shown) of the second leg 92 engage the first slots walls 98a of the release member 86 to retain the fingers 72 within the openings 21 and retain the seal housing assembly 14 on the trocar assembly 12.

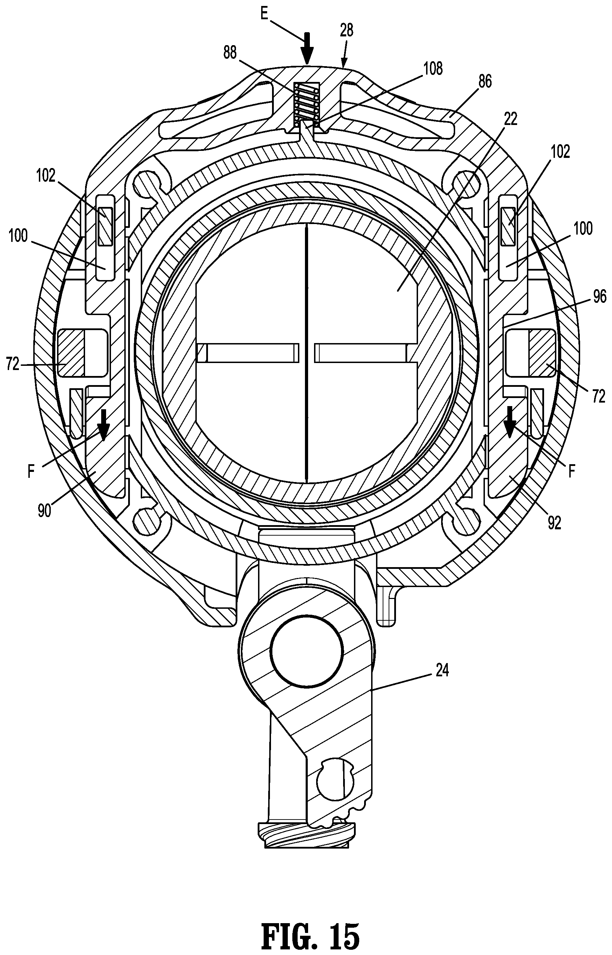

FIG. 15 illustrates the release mechanism 28 in the unlocked position to facilitate separation of the seal housing assembly 14 from the trocar assembly 12. To release the seal housing assembly 14 from the trocar assembly 12, the release member 86 is pressed in the direction indicated by arrow "E" against the urging of biasing member 88 to move the release member 86 to the unlocked position. When the release member 86 is moved to the unlocked position, the slots 96 in the first and second leg members 90 and 92 of the release member 86 are moved into alignment with the fingers 72 of the distal housing portion 48 of the seal housing assembly 14 in the direction indicated by arrows "F". In this position, the fingers 72 of the seal housing assembly 14 can be removed from the openings 21 defined in the proximal housing portion 20 of the trocar assembly 12.

As described above, the seal housing assembly 14 can be attached to or detached from the trocar assembly by simply pressing the release member inwardly and inserting the fingers 72 of the seal housing assembly 14 into the openings 21 of the trocar assembly 12. In aspects of the disclosure, the release member 86 of the access device 10 is positioned at a location angularly spaced from the valves 24 and 26 such that the valves 24 and 26 do not obstruct actuation of the release mechanism 28.

Persons skilled in the art will understand that the devices and methods specifically described herein and illustrated in the accompanying drawings are non-limiting exemplary aspects of the disclosure. It is envisioned that the elements and features illustrated or described in connection with one exemplary embodiment may be combined with the elements and features of another without departing from the scope of the disclosure. Also, one skilled in the art will appreciate further features and advantages of the disclosure based on the above-described aspects of the disclosure. Accordingly, the disclosure is not to be limited by what has been particularly shown and described, except as indicated by the appended claims.

The following examples are illustrative of the techniques described herein.

Example 1. A surgical access device comprising: a trocar assembly including a cannula, a distal housing portion, a proximal housing portion, and a first seal member, the cannula defining a longitudinal axis and a channel that extends through the cannula, the proximal housing portion having an annular configuration and defining openings that extend through the proximal housing portion, the distal housing portion defining a first cavity that communicates with the channel in the cannula, the first seal member received within the first cavity between the distal housing portion and the proximal housing portion; a release mechanism supported on the trocar assembly between the distal housing portion and the proximal housing portion, the release mechanism including a release member and a biasing member, the release member including a first leg and a second leg, each of the first leg and the second leg defining a cam surface, the release member movable in a direction transverse to the longitudinal axis between a locked position and an unlocked position, wherein in the locked position, the cam surfaces of the first leg and the second leg are positioned beneath the openings in the proximal housing portion of the trocar assembly; and a seal housing assembly including a proximal housing portion, a distal housing portion, and a seal assembly, the proximal housing portion and the distal housing portion of the seal housing assembly define a second cavity, the seal assembly received within the second cavity, the distal housing portion including fingers, each of the fingers received within one of the openings of the proximal housing portion of the trocar assembly, wherein receipt of the fingers within the openings causes the release member to move from the locked position to the unlocked position.

Example 2. The surgical access device of Example 1, wherein the biasing member is positioned to urge the release member towards the locked position.

Example 3. The surgical access device of Example 1, wherein each of the first and second legs defines a slot that is configured to allow passage of the fingers when the release member is in the unlocked position

Example 4. The surgical access device of Example 3, wherein the cam surface of each of the first leg and the second leg of the release member define a portion of one of the slots.

Example 5. The surgical access device of Example 3, wherein the release member includes a back span that connects the first leg to the second leg.

Example 6. The surgical access device of Example 5, wherein the back span extends outwardly of the proximal housing portion of the trocar assembly.

Example 7. The surgical access device of Example 6, wherein the proximal housing portion of the trocar assembly includes guide members and each of the first leg and the second leg defines a recess, the guide members received within the recesses to guide movement of the release member between the locked position and the unlocked position.

Example 8. The surgical access device of Example 1, wherein the seal assembly includes a second seal member that is formed of a resilient material and defines an opening for receiving a surgical instrument.

Example 9. The surgical access device of Example 8, wherein the seal assembly is movable within the second cavity in a direction transverse to the longitudinal axis.

Example 10. The surgical access device of Example 9, wherein the seal assembly includes an annular body, and the second seal member is supported within the annular body.

Example 11. The surgical access device of Example 10, wherein the annular body supports resilient strips that extend outwardly of the annular body and engage an inner wall of the proximal housing portion of the seal housing assembly.

Example 12. The surgical access device of Example 2, wherein the proximal housing portion of the trocar assembly includes a post and the biasing member is a coil spring positioned about the post between the release member and the proximal housing portion.

Example 13. A trocar assembly comprising: a cannula, a distal housing portion, a proximal housing portion, and a seal member, the cannula defining a longitudinal axis and a channel that extends through the cannula, the proximal housing portion having an annular configuration and defining openings that extend through the proximal housing portion, the distal housing portion defining a first cavity that communicates with the channel in the cannula, the seal member received within the first cavity between the distal housing portion and the proximal housing portion; and a release mechanism supported on the trocar assembly between the distal housing portion and the proximal housing portion, the release mechanism including a release member and a biasing member, the release member including a first leg and a second leg, each of the first leg and the second leg defining a cam surface, the release member movable in a direction transverse to the longitudinal axis between a locked position and an unlocked position, wherein in the locked position, the cam surfaces of the first leg and the second leg are positioned beneath the openings in the proximal housing portion of the trocar assembly.

Example 14. The trocar assembly of Example 13, wherein the biasing member is positioned to urge the release member towards the locked position.

Example 15. The trocar assembly of Example 13, wherein each of the first and second legs defines a slot that is aligned with one of the openings in the proximal housing portion.

Example 16. The trocar assembly of Example 15, wherein the cam surface of each of the first leg and the second leg of the release member define a portion of one of the slots.

Example 17. The trocar assembly of Example 15, wherein the release member includes a back span that connects the first leg to the second leg.

Example 18. The trocar assembly of Example 17, wherein the back span extends outwardly of the proximal housing portion of the trocar assembly.

Example 19. The trocar assembly of Example 13, wherein the proximal housing portion of the trocar assembly includes guide members and each of the first leg and the second leg defines a recess, the guide members received within the recesses to guide movement of the release member between the locked position and the unlocked position.

Example 20. The trocar assembly of Example 14, wherein the proximal housing portion of the trocar assembly includes a post, and the biasing member is a coil spring positioned about the post between the release member and the proximal housing portion.

## Claims

1. A surgical access device (10) comprising:
a trocar assembly (12) including a cannula (16), a distal housing portion (18), a proximal housing portion (20), and a first seal member (22), the cannula (16) defining a longitudinal axis and a channel that extends through the cannula (16), the proximal housing portion (20) having an annular configuration and defining openings (21) that extend through the proximal housing portion (20), the distal housing portion (18) defining a first cavity (34) that communicates with the channel in the cannula (16), the first seal member (22) received within the first cavity (34) between the distal housing portion (18) and the proximal housing portion (20);
a release mechanism (28) supported on the trocar assembly (12) between the distal housing portion (18) and the proximal housing portion (20), the release mechanism (28) including a release member (86) and a biasing member (88), the release member (86) including a first leg (90) and a second leg (92), each of the first leg (90) and the second leg (92) defining a cam surface (98a, 98b), the release member (86) movable in a direction transverse to the longitudinal axis between a locked position and an unlocked position, wherein in the locked position, the cam surfaces (98a, 98b) of the first leg (90) and the second leg (92) are positioned beneath the openings (21) in the proximal housing portion (20) of the trocar assembly (12); and
a seal housing assembly (14) including a proximal housing portion (20), a distal housing portion (18), and a seal assembly, the proximal housing portion (20) and the distal housing portion (18) of the seal housing assembly (14) define a second cavity (54), the seal assembly (50) received within the second cavity (54), the distal housing portion (18) including fingers (72), each of the fingers (72) received within one of the openings (21) of the proximal housing portion (20) of the trocar assembly (12), wherein receipt of the fingers (72) within the openings (21) causes the release member (86) to move from the locked position to the unlocked position.

2. The surgical access device (10) of claim 1, wherein the biasing member (88) is positioned to urge the release member (86) towards the locked position.

3. The surgical access device (10) according to any of the preceding claims, wherein each of the first and second legs (90, 92) defines a slot (96) that is configured to allow passage of the fingers (72) when the release member (86) is in the unlocked position.

4. The surgical access device (10) of claim 3, wherein the cam surface (98a, 98b) of each of the first leg (90) and the second leg (92) of the release member (86) define a portion of one of the slots (96).

5. The surgical access device (10) according to any of the preceding claims, wherein the release member (86) includes a back span (94) that connects the first leg (90) to the second leg (92).

6. The surgical access device (10) according to any of the preceding claims, wherein the back span (94) extends outwardly of the proximal housing portion (20) of the trocar assembly (12).

7. The surgical access device (10) according to any of the preceding claims, wherein the proximal housing portion (20) of the trocar assembly (12) includes guide members (102) and each of the first leg (90) and the second leg (92) defines a recess (100), the guide members (102) received within the recesses (100) to guide movement of the release member (86) between the locked position and the unlocked position.

8. The surgical access device (10) according to any of the preceding claims, wherein the seal assembly (50) includes a second seal member (76) that is formed of a resilient material and defines an opening for receiving a surgical instrument.

9. The surgical access device (10) according to any of the preceding claims, wherein the seal assembly (50) is movable within the second cavity (54) in a direction transverse to the longitudinal axis.

10. The surgical access device (10) according to any of claims 8 and 9, wherein the seal assembly (50) includes an annular body (75), and the second seal member (76) is supported within the annular body (75).

11. The surgical access device (10) according to any of claims 8-10, wherein the annular body (75) supports resilient strips (78) that extend outwardly of the annular body (75) and engage an inner wall (82) of the proximal housing portion (46) of the seal housing assembly (14).

12. The surgical access device (10) according to any of the preceding claims, wherein the proximal housing portion (20) of the trocar assembly (12) includes a post (108) and the biasing member (88) is a coil spring positioned about the post (108) between the release member (86) and the proximal housing portion (20).
